Europäisches Patentamt

European Patent Office

Office européen des brevets

(19)

(11) Publication number: **0 152 253 B1**

(12) **EUROPEAN PATENT SPECIFICATION**

(45) Date of publication of patent specification: **16.10.91**  (51) Int. Cl.⁵: **C12Q 1/26**, C07F 17/00, C07F 17/02

(21) Application number: **85300733.4**

(22) Date of filing: **04.02.85**

(54) **Method for the determination of ceruloplasmin activity in a sample.**

(30) Priority: **03.02.84 JP 16950/84**

(43) Date of publication of application:
**21.08.85 Bulletin  85/34**

(45) Publication of the grant of the patent:
**16.10.91 Bulletin  91/42**

(84) Designated Contracting States:
**DE FR GB IT**

(56) References cited:
**EP-A- 0 125 137**
**DE-A- 2 027 362**

(73) Proprietor: **Kyowa Medex Co. Ltd.**
**6-1 Ohtemachi Itchome Chiyoda-ku**
**Tokyo(JP)**

(72) Inventor: **Matsumori, Shigeru**
**1188, Shimotogari Nagaizumi-cho**
**Sunto-gun Shizuoka-ken(JP)**
Inventor: **Tatano, Toshio**
**2297-6, Ooka**
**Numazu-shi Shizuoka-ken(JP)**
Inventor: **Shimizu, Yoshiaki**
**127-5, Shimonagakubo Ikeda Nagaizumi-cho**
**Sunto-gun Shizuoka-ken(JP)**

(74) Representative: **Lambert, Hugh Richmond et al**
**D. YOUNG & CO. 10 Staple Inn**
**London, WC1V 7RD(GB)**

## Description

The present invention relates to a method for the determination of ceruloplasmin activity in a sample, and a reagent suitable therefor.

Ceruloplasmin is a copper-containing glycoprotein enzyme capable of oxidising iron from the divalent state to the trivalent state. It is a plasma protein and is usually present in $\alpha_2$-globulin fractions at a concentration of about 0.5% based on total serum weight.

The determination of ceruloplasmin activity is useful in the diagnosis of liver cirrhosis, chromic hepatitis, liver failure, Wilson's disease, etc. Known methods for the assay of ceruloplasmin activity include the single radial diffusion method for the immunologic quantitation of proteins (the SRID method), and colorimetric methods using diamine oxidase. The SRID method is time-consuming (usually more than 24 hours) and the results are not very accurate. The colorimetric method is simpler, but the reaction rate is slow and it still takes 15 to 30 minutes to complete. Not only that, but the colorimetric method cannot be used in automatic assay instruments due to the instability of the reagent.

In accordance with the present invention, it has been found that ceruloplasmin activity in a sample can be determined by reacting the sample with a chromogen in the presence of (a) ferrocene or a ferrocene derivative, (b) a metallocene, or (c) a chelate metal complex containing iron or copper, and measuring the change in absorption due to the formation of pigment in the sample.

Suitable derivatives of ferrocene used in the present invention are: $\alpha$-hydroxyferrocene, benzoylferrocene, 1,1-ferrocene-dicarboxylic acid, acetylferrocene, and N,N-dimethylaminomethylferrocene. Suitable metallocenes are: cobaltocene, nickelocene, zirconocene, and titanocene, whilst suitable chelate metal complexes are: ferrous diethyl-dithiocarbamic acid (DDC-Fe), ferrous acetylacetonate, EDTA-Fe, and copper acetylacetonate. Each may be used in concentrations of from 10 $\mu$M to 1 mM.

Suitable chromogens include: 4-aminoantipyrine (4AA)-phenol system, 4AA-N-ethyl-N-(3-methylphenyl)-N'-acetylethylene-diamine (EMAE) system, N,N-dimethyl-p-phenylenediamine (DPD), methylcarbamoyl-3,7-dimethylamine-10H-phenothiazin (MCDP), and bis[3-bis(4-chlorophenyl)-methyl-4-dimethylaminophenyl]-amine (BCMA), and these may be used at concentrations of from 10 $\mu$M to 1mM.

The reaction is carried out in a buffer solution such as acetate or citrate, at a pH of 4 to 6, preferably 5.

If desired, a surfactant can be added to increase solubility or remove turbidity due, for example, to high concentrations of fat in the serum sample. Suitable surfactants are Triton X-100, polyoxyethylenelaurylether and sodium laurylsulfate. (Triton is a Registered Trade Mark.)

The change in absorbance of the reaction solution due to the formed pigment is measured after a period of from 1 to 15 minutes, preferably at the wavelength of maximum absorption of the pigment, and usually in the range 600 to 800 nm. Ceruloplasmin activity can then be determined by reference to a standard curve.

To illustrate the invention, 1 mg of each of the compounds indicated in Table 1 and 1.65 mg of MCDP were added to 100 ml quantities of acetate buffer to prepare a series of reagent solutions. 3 ml of each reagent solution were then incubated at 37°C. 30 $\mu$l of serum was added to each solution and the change in absorption ($\Delta$ E) at 663 nm was measured for 1 minute, 2 minutes after the reaction started. The results are shown in Table 1.

TABLE 1

|  | $\Delta E \times 10^3/min$ |
|---|---|
| control | 1 |
| ferrocene | 63 |
| $\alpha$-hydroxyferrocene | 61 |
| benzoylferrocene | 12 |
| 1,1-ferrocene-dicarboxylic acid | 60 |
| acetylferrocene | 30 |
| N,N-dimethylaminomethylferrocene | 45 |
| cobaltocene | 3 |
| titanocene | 3 |
| zirconocene | 2 |
| nickelocene | 2 |
| ferrous diethyl-dithio-carbamic acid | 24 |
| ferrous acetylacetonate | 3 |
| EDTA-Fe | 2 |
| copper acetylacetonate | 3 |

The above procedure was repeated using 20 mg quantities of 4AA-EMAE, DPD and MCDP each with 1 mg of ferrocene to obtain the results shown in Table 2.

TABLE 2

| Chromogen | No addition of ferrocene $\Delta$ $E \times 10^3/min$ | Addition of ferrocene $\Delta$ $E \times 10^3/min$ |
|---|---|---|
| 4AA-EMAE | 1 | 7 |
| DPD | 3 | 7 |
| MCDP | 1 | 63 |

The results obtained are not influenced by other substances present in serum because the reaction speed is very high, so that the ceruloplasmin activity can be measured very quickly and with high accuracy.

The invention is further illustrated by the following example.

EXAMPLE

To 100 ml of a solution (pH 5.3) having the composition:

| 0.2M-acetate buffer | 11.3 |
|---|---|
| 0.2M-sodium acetate solution | 38.7 |
| Triton X-100 | 0.1 |
| Water | 50 |

were added 1 mg of ferrocene and 1.65 mg of MCDP. 3 ml of the reagent solution was then poured into a cell and incubated at 37° C for 5 minutes. To the reagent solution were then added 30 $\mu$l of serum and the change in absorption ($\Delta E$) of the reaction solution at 663 nm was measured for 1 minute, two minutes after the reaction started. The results are shown in Table 3.

The same procedure was repeated, but using 10 mg of N,N-dimethylaminomethylferrocene (Test No. 2), 10 mg of titanocene (Test No. 3), 10 mg of nickelocene (Test No. 4), 7.5 mg of DDC-Fe (Test No. 5), or 10 mg of copper acetylacetonate (Test No. 6) instead of ferrocene. Finally in Test No. 7, 10 mg of N,N-dimethylaminomethylferrocene was used instead of ferrocene and 12.3 mg of oxalate of DPD instead of MDCP were used to obtain the results in Table 3.

3

TABLE 3

| Test No. | Sample 1 | | | Sample 2 | | |
|---|---|---|---|---|---|---|
| | A | U/ml | S | A | U/ml | S |
| 1 | 63 | 79.0 | 30.2 | 110 | 138.0 | 52.8 |
| 2 | 45 | 56.0 | 30.2 | 78 | 98.0 | 52.3 |
| 3 | 3 | 3.7 | 30.0 | 5 | 6.3 | 50.0 |
| 4 | 2 | 2.5 | 28.0 | 4 | 5.0 | 56.0 |
| 5 | 24 | 30.0 | 31.2 | 41 | 51.0 | 53.3 |
| 6 | 3 | 3.8 | 30.0 | 5 | 6.3 | 50.0 |
| 7 | 15 | 19.0 | 30.0 | 26 | 33.0 | 52.0 |
| A: $\Delta$ E/min x $10^3$ | | | | | | |
| S: Content of ceruloplasmin (mg/dl) | | | | | | |
| U: International unit | | | | | | |

Also in accordance with this invention there is provided a colorimetric reagent for the determination of ceruloplasmin activity in a sample comprising both a chromogen as defined and the said ferrocene or ferrocene derivative, metallocene or said iron- or copper-containing chelate metal complex.

## Claims

1. A method for determining ceruloplasmin activity in a sample which comprises reacting the sample with a chromogen in the presence of (a) ferrocene or a ferrocene derivative, (b) a metallocene, or (c) a chelate metal complex containing iron or copper, thereby to form a pigment, and photometrically measuring the change in absorption of the sample due to the formation of said pigment.

2. A method according to claim 1, wherein there is used a ferrocene derivative selected from $\alpha$-hydroxyferrocene, benzoylferrocene, 1,1-ferrocene-dicarboxylic acid, acetylferrocene, and N,N-dimethylaminomethylferrocene.

3. A method according to claim 1, wherein there is used a metallocene selected from cobaltocene, nickelocene, zirconocene, and titanocene,

4. A method according to claim 1, wherein there is used a chelate metal selected from ferrous diethyl-dithiocarbamic acid, ferrous acetylacetonate, EDTA-Fe, and copper acetylacetonate.

5. A method according to any one of claims 1 to 4, wherein there is used as said chromogen: 4-aminoantipyrine (4AA)-phenol system, 4AA-N-ethyl-N-(3-methylphenyl)-N'-acetylethylene-diamine (EMAE) system, N,N-dimethyl-p-phenylenediamine (DPD), methylcarbamoyl-3,7-dimethylamine-10H-phenothiazin (MCDP), or bis[3-bis(4-chlorophenyl)methyl-4-dimethylaminophenyl]amine (BCMA).

6. A method according to any one of claims 1 to 5, wherein the reaction is carried out in a buffer solution at a pH of 4 to 6.

7. A method according to any one of claims 1 to 6, wherein said measurement is carried out at the wavelength of maximum absorption of the chromogen.

8. A reagent for the determination of ceruloplasmin activity in a sample comprising (A) (a) ferrocene or a ferrocene derivative, (b) a metallocene, or (c) a chelate metal complex containing iron or copper, and (B) a chromogen selected from 4-aminoantipyrine (4AA)-phenol system, 4AA-N-ethyl-N-(3-methylphenyl)-N'-acetylethylene-diamine (EMAE) system, N,N-dimethyl-p-phenylenediamine (DPD), methylcarbamoyl-3,7-dimethylamine-10H-phenothiazin (MCDP), or bis[3-bis(4-chlorophenyl)methyl-4-dimethylaminophenyl]amine (BCMA).

9. A reagent according to claim 8, wherein component (A) is
α-hydroxyferrocene
benzoylferrocene
1,1-ferrocene-dicarboxylic acid
acetylferrocene
N,N-dimethylaminomethylferrocene
cobaltocene
titanocene
zirconocene
nickelocene
ferrous diethyl-dithio-carbamic acid
ferrous acetylacetonate
EDTA-Fe
or copper acetylacetonate.

## Revendications

1. Procédé pour mesurer- l'activité de la céruloplasmine dans un échantillon, consistant à faire réagir l'échantillon avec un agent chromogène en présence de (a) le ferrocène ou un dérivé du ferrocène, (b) un métallocène ou (c) un complexe métallique de chélation contenant du fer ou du cuivre, pour former ainsi un pigment, et à mesurer par photométrie la variation d'absorption de l'échantillon, due à la formation dudit pigment.

2. Procédé selon la revendication 1, dans lequel on utilise un dérivé du ferrocène choisi parmi α-hydroxyferrocène, benzoylferrocène, acide 1,1-ferrocènedicarboxylique, acétylferrocène et N,N-diméthyaminométhylferrocène.

3. Procédé selon la revendication 1, dans lequel on utilise un métallocène choisi parmi cobaltocène, nickelocène, zirconocène et titanocène.

4. Procédé selon la revendication 1, dans lequel on utilise un chélate métallique choisi parmi diéthyldithiocarbamate ferreux, acétylacétonate ferreux, EDTA-Fe et acétylacétonate de cuivre.

5. Procédé selon l'une quelconque des revendications 1 à 4, dans lequel on utilise; comme agent chromogène: le système 4-aminoantipyrine (4AA)-phénol, le système 4AA-N-éthyl-N-(3-méthylphényl)-N'-acétyléthylènediamine (EMAE), la N,N-diméthyl-p-phénylènediamine (DPD), la méthylcarbamoyl-3,7-diméthylamino-10H-phénothiazine (MCDP) ou la bis[3-bis(4-chlorophényl)méthyl-4-diméthylaminophényl]amine(BCMA).

6. Procédé selon l'une quelconque des revendications 1 à 5, dans lequel la réaction est effectuée en solution tampon à un pH de 4 à 6.

7. Procédé selon l'une quelconque des revendications 1 à 6, dans lequel ladite mesure est effectuée à la longueur d'onde du maximum d'absorption de l'agent chromogène.

8. Réactif pour la mesure de l'activité de la céruloplasmine dans un échantillon, comprenant (A) (a) le ferrocène ou un dérivé-du ferrocène, (b) un métallocène ou (c) un complexe métallique de chélation contenant du fer ou du cuivre, et (B) un agent chromogène choisi parmi: le système 4-aminoantipyrine (4AA)-phénol, le système 4AA-N-éthyl-N-(3-méthylphényl)-N'-acétyléthylènediamine (EMAE), la N,N-diméthyl-p-phénylènediamine (DPD), la méthylcarbamoyl-3,7-diméthylamino-10H-phénothiazine (MCDP) ou la bis[3-bis(4-chlorophényl)méthyl-4-diméthylaminophényl]amine(BCMA).

9. Réactif selon la revendication 8, dans lequel le composant (A) est:
α-hydroxyferrocène,
benzoylferrocène,
acide 1,1-ferrocène-dicarboxylique,
acétylferrocène,
N,N-diméthylaminométhylferrocène,

cobaltocène,
nickelocène,
zirconocène,
titanocène,
diéthyl-dithiocarbamate ferreux,
acétylacétonate ferreux,
EDTA-Fe,
acétylacétonate de cuivre.

## Patentansprüche

1. Verfahren zur Bestimmung der Ceruloplasmin-Aktivität in einer Probe, umfassend die Umsetzung der Probe mit einem Chromogen in Gegenwart von (a) Ferrocen oder einem Ferrocen-Derivat, (b) einem Metallocen, oder (c) einem Eisen oder Kupfer enthaltenden Metall-Chelat-Komplex, wobei ein Pigment erzeugt wird, und photometrische Messung der Änderung der Absorption der Probe infolge der Entstehung des Pigments.

2. Verfahren nach Anspruch 1, wobei ein Ferrocen-Derivat, ausgewählt aus $\alpha$-Hydroxyferrocen, Benzoylferrocen, 1,1-Ferrocen-dicarbonsäure, Acetylferrocen und N,N-Dimethylaminomethylferrocen verwendet wird.

3. Verfahren nach Anspruch 1, wobei ein Metallocen, ausgewählt aus Cobaltocen, Nickelocen, Zirkonocen und Titanocen verwendet wird.

4. Verfahren nach Anspruch 1, wobei ein Metall-Chelat, ausgewählt aus Eisen-diethyldithiocarbaminsäure, Eisen-acetylacetonat, EDTA-Fe und Kupfer-Acetylacetonat verwendet wird.

5. Verfahren nach einem der Ansprüche 1 bis 4, wobei als Chromogen das 4-Aminoantipyrin (4AA)-Phenol-System, 4AA-N-Ethyl-N-(3-methylphenyl)-N'-acetylethylen-diamin (EMAE)-System, N,N-Dimethyl-p-phenylendiamin (DPD), Methylcarbamoyl-3,7-dimethylamin-10H-phenothiazin (MCDP) oder Bis[3-bis(4-chlorphenyl)-methyl-4-dimethylaminophenyl]-amin (BCMA) verwendet wird.

6. Verfahren nach einem der Ansprüche 1 bis 5, wobei die Reaktion in einer Pufferlösung bei einem pH-Wert von 4 bis 6 durchgeführt wird.

7. Verfahren nach einem der Ansprüche 1 bis 6, wobei die Messung bei der Wellenlänge der maximalen Absorption des Chromogens durchgeführt wird.

8. Reagenz zur Bestimmung der Ceruloplasmin-Aktivität in einer Probe, umfassend (A) (a) Ferrocen oder ein Ferrocen-Derivat, (b) ein Metallocen, oder (c) einen Eisen oder Kupfer enthaltenden Metall-Chelatkomplex, und (B) ein Chromogen, ausgewählt aus dem 4-Aminoantipyrin (4AA)-Phenol-System, 4AA-N-Ethyl-N-(3-methylphenyl)-N'-acetylethylen-diamin (EMAE)-System, N,N-Dimethyl-p-phenylendiamin (DPD), Methylcarbamoyl-3,7-dimethylamin-10H-phenothiazin (MCDP), oder Bis[3-bis(4-chlorphenyl)-methyl-4-dimethylaminophenyl]-amin (BCMA).

9. Reagenz nach Anspruch 8, wobei Komponente (A)
$\alpha$-Hydroxyferrocen,
Benzoylferrocen,
1,1-Ferrocen-dicarbonsäure,
Acetylferrocen,
N,N-Dimethylaminomethylferrocen,
Cobaltocen,
Titanocen
Zirkonocen
Nickelocen
Eisen-diethyldithiocarbaminsäure,
Eisen-acetylacetonat,
EDTA-Fe oder

Kupfer-acetylacetonat
ist.